# EUROPEAN PATENT SPECIFICATION

(11) **EP 0 495 064 B1**
(45) Date of publication and mention of the grant of the patent: **11.07.2001**
(21) Application number: 91914581.3
(22) Date of filing: 09.08.1991
(51) Int. Cl.: C12N 15/29, C07K 14/42, C12N 15/70, C12Q 1/68, A61K 38/02

(54) **BIRCH POLLEN ALLERGEN P14 FOR DIAGNOSIS AND THERAPY OF ALLERGIC DISEASES**
BIRKENPOLLENALLERGEN P14 ZUR DIAGNOSE UND THERAPIE VON ALLERGISCHEN KRANKHEITEN
ALLERGENE P14 AU POLLEN DE BOULEAU SERVANT AU DIAGNOSTIC ET A LA THERAPIE DES MALADIES ALLERGIQUES

(30) Priority: 13.08.1990 AT 168590; 11.04.1991 US 683832
(43) Date of publication of application: 22.07.1992
(73) Proprietor: "BIOMAY" Produktions- und Handelsgesellschaft m.b.H., A-4020 Linz (AT)
(72) Inventor: VALENTA, Rudolf, A-2604 Theresienfeld (AT); DUCHENE, Michael, A-1090 Vienna (AT); PETTENBURGER, Karin, A-1040 Vienna (AT); BREITENBACH, Michael, A-1120 Vienna (AT); KRAFT, Dietrich, A-1170 Vienna (AT); RUMPOLD, Helmut, A-1180 Vienna (AT); SCHEINER, Otto, A-2364 Mariaenzersdorf (AT)
(74) Representative: Itze, Peter, Dipl.-Ing.
(86) International application number: EP9101513
(87) International publication number: WO9203551

(56) References cited:
- DE-A- 3 147 763
- Clinical Experimental Allergy, volume 20, 1990, suppl. 1, meeting 8-11 July 190, S. d'Abusco et al.: "Characterizstion of cDNA for Parietaria pollen allergens", page 48, see abstract OP52 (cited in the application)
- Biochimica et Biophysics Acta, volume 967, 1988, Elsevier, U. Lindberg et al.: "The use of poly(L-proline)-sepharose in the isolation of profilin and profilactin complexes", pages 391-400, see the whole document
- Int. Arch. Allergy Appl. Immunol., volume 98, 1988, H. Breiteneder et al.: "Isolation and characterization of messenger RNA from male inflorescences and pollen of the white birch (Betula verrucosa), pages 19-24, see page 21, left-hand column, "Immunoblotting of aqueous BV pollen extracts"
- The EMBO Journal, volume 8, no. 7, July 1989, (Eynsham, Oxford, GB) H. Breiteneder et al.: "The gene coding for the major birch pollen allergen Betvl, is highly homologous to a pea disease resistance response gene", pages 1935-1938, see the whole article
- Science, volume 253, 2 August 1991, R. Valenta et al.: "Identification of profilin as a novel pollen allergen; IgE autoreactivity in sensitized individuals", pages 557-560, see the whole article
- Allergy, volume 44, no. 6, August 1989, E. Jarolim et al.: "IgE and IgG antibodies of patients with allergy to birch pollen as tools to define the allergen profile of Betula verrucosa", pges 385-395, see the whole article
- Allergy, volume 45, no. 6, August 1990, T. Birkner et al.: "Evaluation of immunotherapy-induced changes in specific IgE, IgG and IgG subclasses in birch pollen allergic patients by means of immunoblotting", pages 418-426, see the whole article

## Description

### 1. FIELD OF THE INVENTION

The invention provides recombinant DNA molecules which code for polypeptides, and the polypeptides per se, that have at least one epitope of a P14 pollen allergen of a tree of the order Fagales, particularly birch (Betula verrucosa), or the entire P14 allergen protein, and exhibit the same or similar antigenicity as a P14 allergen. The invention also provides replicable microbial expression vehicles and microorganisms for use in processes for producing such allergenic polypeptides. Methods are provided for purification of P14 allergen as well as for the diagnosis and therapy of allergic diseases using the synthetic polypeptides of the invention.

### 2. BACKGROUND OF THE INVENTION

In the springtime large parts of the populations of Central, Eastern and Northern Europe, America and Australia suffer from allergic symptoms (rhinitis, conjunctivitis, dermatitis and pollen asthma). Proteins which can be isolated from pollen of trees of the order Fagales, in particular from pollen of birch, alder, hazel, hornbeam and oak, are responsible for most of these allergic symptoms (1).

At least 10% of the population suffers from pollen allergies at various times and to varying extent. These allergies are mediated by IgE antibodies which react with pollen proteins. The possibility exists for a therapy for pollen allergies by hyposensitization, i.e., by the regular and slowly increasing administration of the proteins producing the allergy.

Diagnostic methods for allergic diseases, such as RIA (radioimmunoassay), IRMA (immuno-radiometric assay), RAST (radio-allergosorbent test), ELISA (enzyme-linked immunosorbent assay), magnetic allergoabsorbent test, immunoblots, LIA (luminescence immunosay), Histamine release assays and others depend greatly upon the availability of pure allergens. Protein extracts from pollen isolated from natural sources are difficult to standardize because preparations vary from batch to batch. For example, they may contain unwanted constituents, and/or certain proteins may be lost in the extraction procedure and be missing from the final separation (2). Clearly, diagnostic tests which employ well defined allergens that can be reproducibly prepared would be superior to tests which employ raw pollen extracts with an insufficiently defined mixture of allergens and other components. Recombinant DNA production of allergenic polypeptides, or allergenic fragments thereof, would allow more reproducible preparations of allergens of defined content for standardized diagnostic and therapeutic methods.

Allergens may be purified to homogenity from pollen by known protein/chemical methods, for example, by means of affinity chromatography (3). These methods are relatively costly and require pollen as an ill-defined source which cannot be standardized. It would, therefore, be cheaper and more efficient to use recombinant DNA methods to produce an allergenic protein, or fragments of that protein.

Hyposensitization has proved to be an effective therapy in allergic diseases. This therapy consists of parenteral or oral administration of allergens in increasing doses over a fairly long period of time. Like diagnostic methods, it requires pure and well defined allergens. The use of purified recombinant allergens or synthetic peptides would greatly reduce the risk of sensitizing patients to unwanted components.

In Abstract OP 52 on page 48 of Supplement 1 of Clinical and Experimental Allergy, Vol.20, which refers to a presentation having taken place 8 - 11 July, 1990, a 14kDA allergen of Parietarium judaica is described generally without showing a sequence.

### 3. SUMMARY OF THE INVENTION

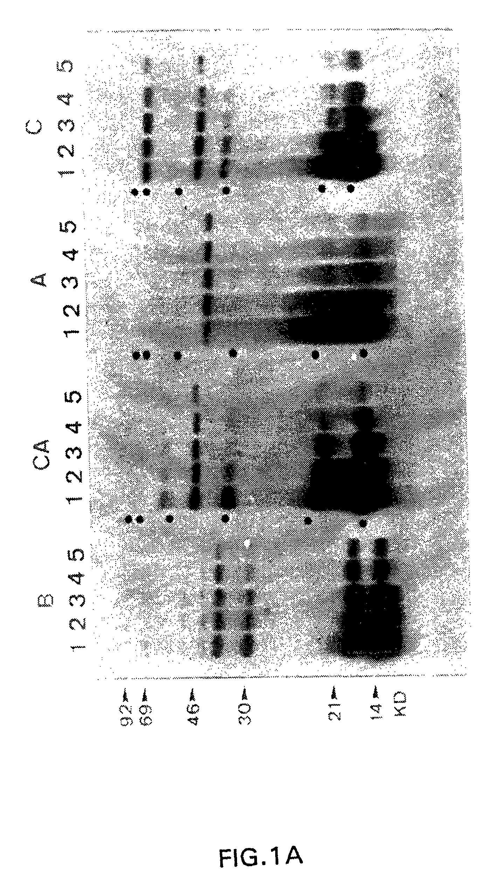

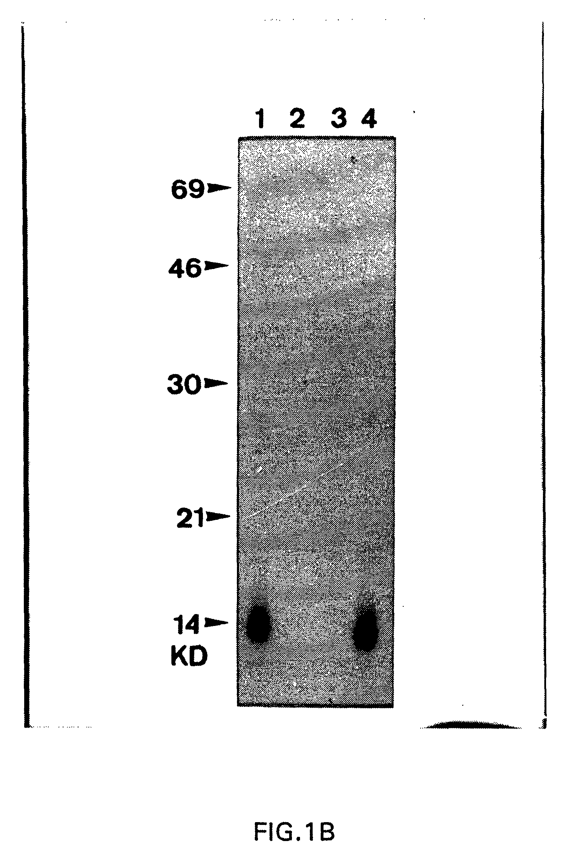

This invention concerns pollen allergens, for example, of white birch (Betula verrucosa), called P14. These pollen allergens are immunologically closely related to allergens which occur in pollen of far distantly related plant species, particularly in trees of the Fagales order (birch, alder, hazel, hornbeam and oak), in grasses and weeds. The cross-reactivity of IgE antibodies of patients to these pollen allergens is illustrated in FIGS. 1A and 1B.

The present invention provides a recombinant DNA molecule consisting of the sequence of SEQ. ID No.2. The invention provides therefore the complete cDNA sequence of a P14 allergen and hence the complete deduced amino acid sequence. Additionally, the invention includes a recombinant DNA molecule, comprising DNA that is complementary to a nucleic acid molecule that hybridizes under stringent conditions to a DNA molecule consisting of the nucleid acid sequence of SEQ ID NO:2 and which encodes a protein that (a) binds to IgE antibodies in serum of an individual allergic to an allergen consisting of the amino acid sequence of SEQ ID NO:3 and (b) binds to poly-(L-proline), wherein said stringent conditions are: at 55°C, a salt concentration of 150 mM NaCl and 15 mM Na₃citrate x 2H₂O, at pH 7,0 and with a sodium dodecyl sulfate at a concentration of 0,1% weight/volume. This nucleotide sequence can be expressed as a P14 allergen, or as a polypeptide which comprises at least one epitope thereof.

As concerns their IgE binding, pollens of birch, alder, hazel, hornbeam, oak, grasses and weeds possess similar allergens as the P14 allergen. The present invention therefore relates not only to a P14 allergen of birch, but as well to P14 pollen allergens of other species which are coded by DNA sequences that are able to hybridize with the nucleotide sequence of a birch P14 allergen under stringent conditions or can be derived from such polypeptide be degeneracy of the genetic code. Hybridization of a polynucleotide with another polynucleotide under stringent conditions requires at least a 60 % identity between such polynucleotides at the nucleic acid level.

Such stringent conditions entail washing of hybridized nitrocellulose filters as follows:
a) For DNA/DNA and DNA/RNA hybridizations: A temperature of 55°C, a salt concentration of 150 mM NaCl and 15 mM Na₃citrate x 2 H₂O, at pH 7,0, and SDS (Sodium Dodecyl Sulfate) detergent at a concentration of 0,1 % (w/v).
b) For oligodeoxynucleotides/DNA hybridizations: A temperature of 55°C, a salt concentration of 1M NaCl and 10mM Na₃citrate x 2 H₂O, at pH 7,0, and SDS (Sodium Dodecyl Sulfate) detergent at a concentration of 0,5 % (w/v). In this context "oligodeoxynucleotide" refers to an oligomer of a single stranded DNA of up to 100 nucleotides in length.

In addition, this invention provides a replicable microbial expression vehicle, which directs expression of a DNA as described above to produce the protein.

This invention also concerns compositions containing synthetic polypeptides which exhibit the antigenicity of parts or of the whole of a birch P14 allergen or of allergens of other plants which, because of a high degree (at least 50%) of amino acid homology, exhibit antigenic cross-reactivity to parts or to all of a birch P14 allergen, i.e., antibodies or cellular antigen binding sites which are actually directed to birch P14 allergen are likewise able to bind to these molecules. These synthetic polypeptides include fusion and nonfusion polypeptides which contain a polypeptide portion that possesses the antigenicity of a part or of all of a P14 allergen. The method for preparing such synthetic polypeptides comprises the steps of culturing of prokaryotic or eukaryotic host cells which contain an expression plasmid described above and purification of the synthetic polypeptide(s) from the culture.

The term "synthetic" here alternatively includes polypeptides which are prepared by cloning and expression of the nucleotide sequences described here or by chemical synthetis of polypeptides encoded by these nucleotide sequences.

The synthetic polypeptides which are produced by using the recombinant DNA molecule according to this invention exhibit antigenicity the same as or similar to the native allergen. As shown below, a cDNA clone coding for a birch P14 can be used to produce a nonfusion polypeptide which reacts with IgE in the sera of allergic persons. It is therefore possible to use this polypeptide as an antigen in diagnostic tests (such as RAST, ELISA, Immunoblots and others known in the art and referred to above) and as a component of therapeutic agents in hyposensitization therapy.

In particular, the synthetic allergens can be used as diagnostic reagents in vitro and in vivo, since their antigenicity corresponds to that of the native P14 pollen allergens and they are therefore able to bind IgE in sera of persons suffering from P14 pollen allergy.

It is therefore one of the objects of the present invention to provide a method for the preparation of pollen allergens, in particular for birch P14 allergens, so as to have this family of allergens available for diagnostic tests for detection of the corresponding allergy and, alternatively, for hyposensitization therapy.

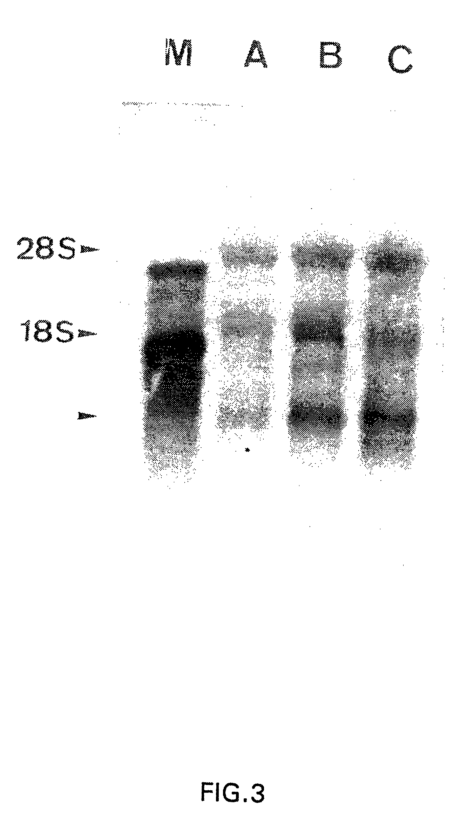

In addition, as shown below, birch P14 cDNA was expressed in two prokaryotic expression systems in Escherichia coli, and the IgE-binding capacity of the expressed polypeptides, a fusion protein and a nonfusion protein, was demonstrated. This expression can also be achieved in any other microorganism (e.g., eukaryotic cells). The IgE-binding capacity was also demonstrated for a partial sequence which was expressed, using lambda gt11, as a β-galactosidase fusion protein. In this way it was demonstrated that this partial sequence represents at least one IgE-binding epitope. In addition, it may be concluded from the results of IgE immunoblots, cross-inhibition tests, clinical tests and Northern (RNA) blots (4-9) (FIGS. 1A, 1B and 3) that homologous IgE-binding polypeptides exist in the pollen of closely related trees of the order Fagales and for distantly related pollen producing plants. For this reason this invention provides polypeptides which exhibit the same or similar antigenicity as the related P14 pollen allergens of birch, alder, hazel, hornbeam, oak, grasses and weeds.

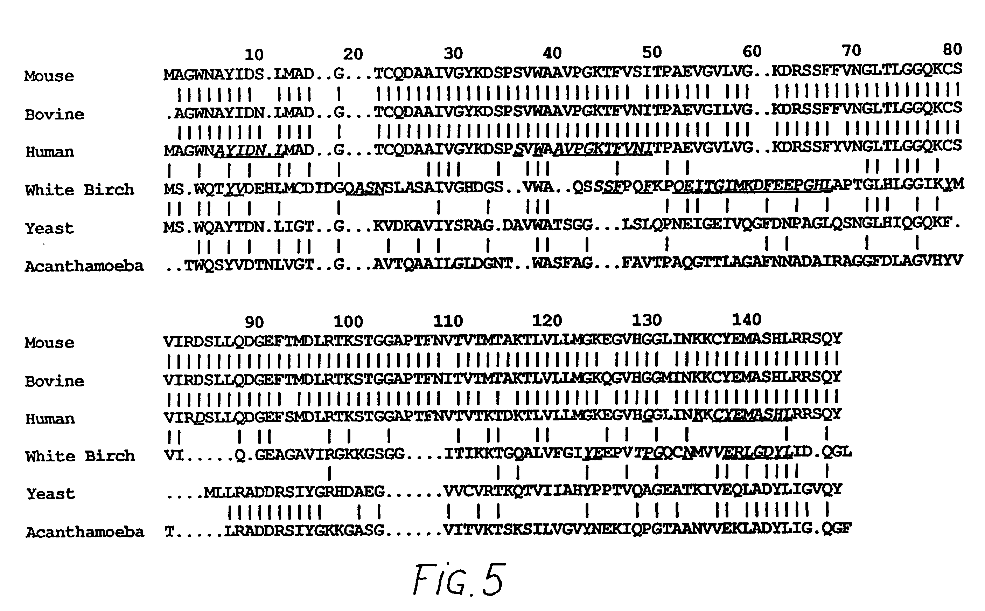

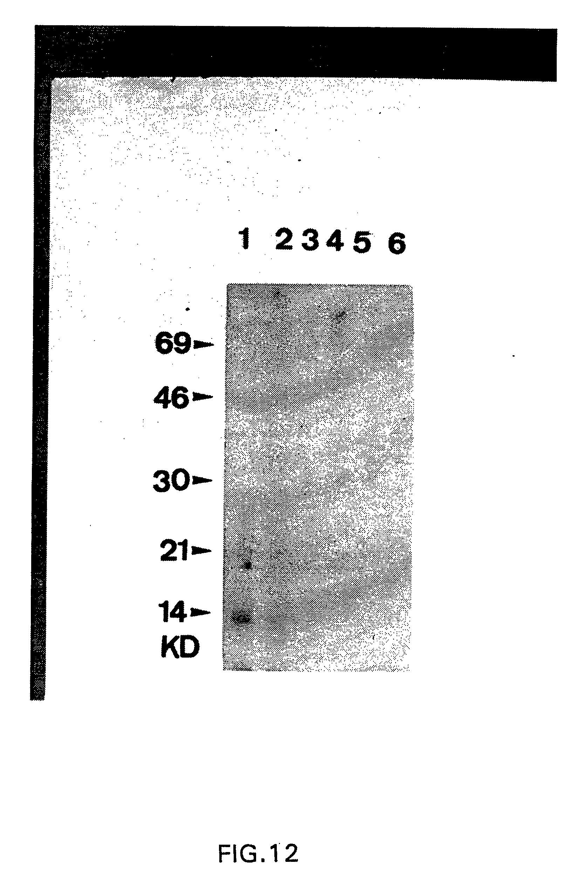

A computer search in the available sequence data banks (EMBL, MIPSX, Swissprot) for proteins whose sequences share homology with birch P14 revealed a significant homology between P14 and a cytoskeletal protein (profilin) which is present in a variety of eukaryotes (10-14) (FIG. 5). This homology raises the possibility of the cross-reactivity of IgE antibodies of patients with human profilin. This autoreactivity has been demonstrated (FIG. 12).

In this way, a molecular system is provided which permits testing the hypothesis: whether autoimmune mechanisms play a role in allergic and atopic diseases. Initial data show that patients whose IgE antibodies react with P14 represent a group that suffers from allergic symptoms during a large part of the year and who do not respond satisfactorily to immunotherapy or conventional therapy. It follows from this that P14, or recombinant or chemically synthesized IgE-binding polypeptides with sequences that match the sequence deduced from P14 cDNA, can be used to characterize a certain group of multivalent allergics as well as a prognostic markers for hyposensitization therapy.

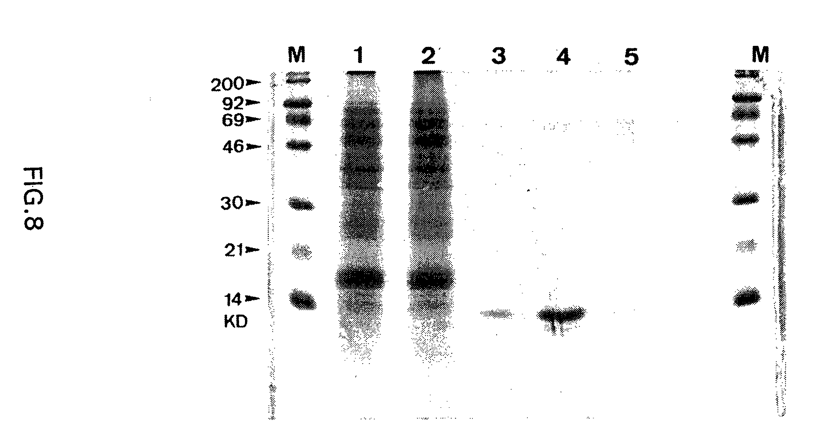

In addition, this invention presents an efficient method for the production and purification of pollen protein as well as of recombinant or synthetic P14 polypeptide or allergenic fragments thereof. The purification method is based on the affinity of profilin polypeptides for poly(L-proline) (15, 16), and the present inventors' showing of homology between profilins and P14 allergens. Since the binding of pollen protein and of recombinant P14 to poly(L-proline) has been shown herein (FIGS. 8, 9 and 10), a method is thereby provided for immobilizing (and affinity separation of) this allergen. Thus, certain diagnostic tests can be set up (for example, poly(L-proline) may be used instead of an antibody for binding profilin in ELISAs). Likewise, forms of therapy are possible which by means of poly(L-proline) bind P14 and analogous polypeptide allergens. Since there are indications that patients who suffer from autoimmune diseases form antibodies against P14, this polypeptide or homologous polypeptides could be used for diagnosis or therapy of these diseases.

### 4. BRIEF DESCRIPTION OF THE FIGURES

The following figures and description aid in understanding the field and scope of the invention.

FIG. 1A: IgE immunoblot: Pollen proteins from birch (B), hornbeam (CA), alder (A) and hazel (C) were separated by means of a 12.5% polyacrylamide electrophoresis and blotted on nitrocellulose. The nitrocellulose was cut into strips (1-5) which were incubated with dilutions of a serum (1:5, 1:10, 1:20, 1:40, 1:80, respectively) of a selected patient whose IgE antibodies recognized most important birch pollen allergens. Arrows and stars indicate molecular weights. Bound serum IgE was detected by means of an autoradiograph of ¹²⁵I-labeled antihuman IgE antibodies of rabbit bound thereto. The IgE-binding proteins of birch, alder, hazel and hornbeam matched one another, which demonstrates the similarity of the antigens.

FIG. 1B: IgE immunoblot inhibition: mugwort profilin that had been purified by poly(L-proline) affinity chromatography had been blotted on nitrocellulose after polyacrylamide gel electrophoresis. The 1:10 dilution of the serum from a patient allergic to birch profilin was pre-incubated in lane 1 with control proteins from E. coli, in lane 2 with recombinant birch profilin, in lane 3 with purified profilin from Phleum pratense (grass) and in lane 4 with buffer (negative control) and used for detection of mugwort parofilin. Binding of patients' IgE to mugwort profilin can be blocked with recombinant birch profilin and purified grass profilin demonstrating common IgE binding properties of these related proteins. In the control lanes 1 and 4 binding of patients' IgE to mugwort profilin occurs.

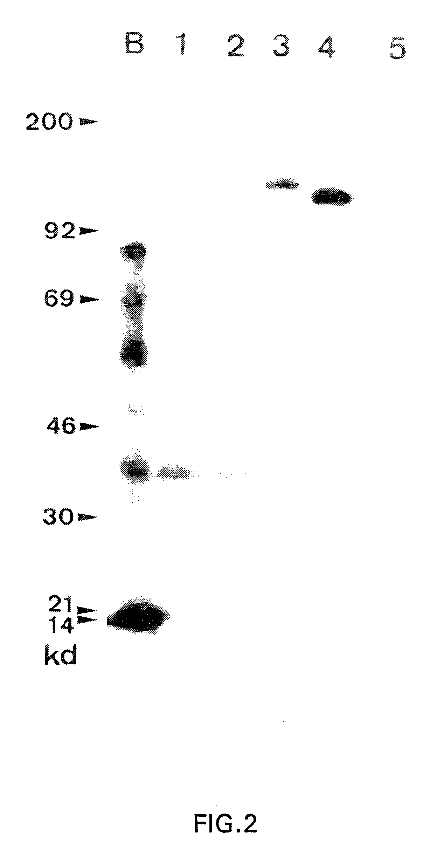

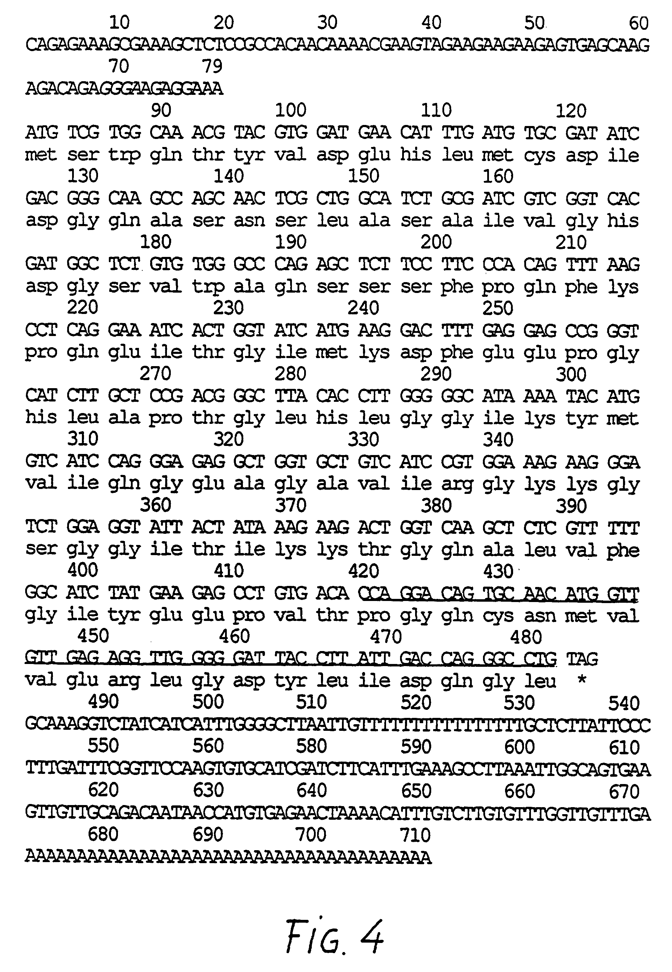

FIG. 2: IgE immunoblot: Proteins were separated by means of a 7.5% polyacrylamide gel electrophoresis and blotted on nitrocellulose. Lane B: birch pollen proteins; lane 1: proteins from E. coli Y1089 (lysogenic host); lane 2: proteins of E. coli Y1089 inoculated with the lambda gtll phage without insert; lane 3: proteins from E. coli Y1089 inoculated with a recombinant phage containing a birch pollen derived cDNA encoding an IgE binding polypeptide (positive control); lane 4: proteins from E. coli Y1089 inoculated with recombinant phages which contain the 3'-portion of P14 cDNA which codes for an IgE-binding epitope (as underlined in FIG. 4); lane 5: proteins from yeast (Saccharomyces cerevisiae). Recombinant β-galactosidase fusion proteins with IgE-binding capacity whose molecular weights were between 115 and 130 kD (lanes 3 and 4) were detected with ¹²⁵I-labeled antihuman IgE antiserum from rabbit. No comparable IgE binding takes place in lanes 1, 2 and 5, while lane B shows the patient's IgE-binding profile with birch pollen proteins.

FIG. 3: Northern (RNA) blot: Ten *µ*g pollen RNA of alder (lane A), birch (lane B) and hazel (lane C) and as a marker RNA of E. coli (lane M) were blotted on nitrocellulose. The part of the P14 cDNA underlined in FIG. 4 hybridizes with pollen mRNA of alder, birch and hazel and under stringent conditions (0.75 x SSC, 0.1% SDS, 50°C) produces a signal at 800 bases (indicated by an arrow). The position of ribosomal bands is indicated by "28S" and "18S".

FIG. 4: cDNA sequence of birch P14. The coding region begins with ATG (nucleotides 80-82) and ends with the stop codon TAG (nucleotides 479-481). The deduced amino acid sequence is illustrated under the DNA sequence. The P14 sequence, which within a fusion protein is able to bind IgE of patients and therefore represents at least one epitope, is shown underscored (see Section 5.4).

FIG. 5: Comparison of the derived amino acid sequence of birch P14 with the amino acid sequences of profilins of human (13), calf (14), mouse (12), yeast (11) and Acanthamoeba (10). Identical amino acid residues are marked. The percentage of identical amino acid residues between P14 protein of birch and homologs amounts to 30% for human protein, 28% for homologous proteins of calf and mouse, 26% for yeast protein and 25% for Acanthamoeba protein.

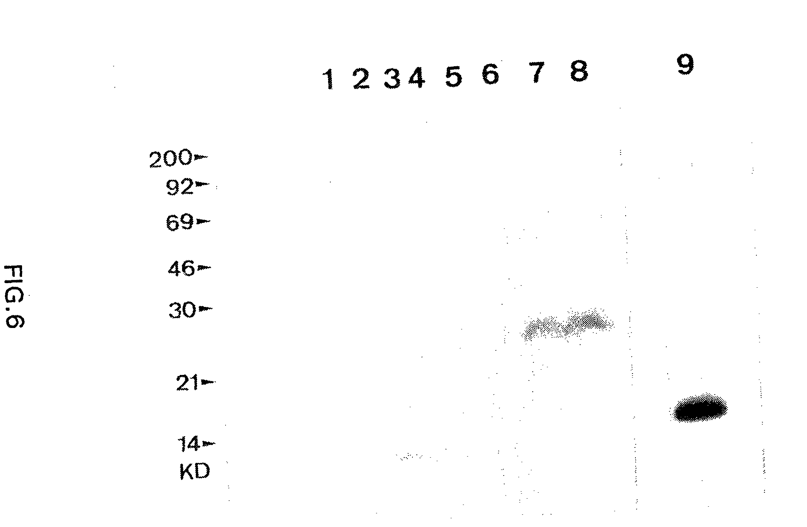

FIG. 6: Western (protein) blot of a polyacrylamide gel probed with IgE antibodies of patients with tree pollen allergy. Lane 1: proteins of E. coli JM105 without any plasmid; lane 2: proteins of E. coli JM105 with the plasmid pKK223-3 without an insert; lanes 3 and 4: proteins of E. coli JM105 with that plasmid derived from pKK223-3 which expresses the P14 protein of the inserted cDNA as nonfusion protein; lane 5: E. coli AR58 proteins; lane 6: E. coli AR58 with the plasmid pEXB without an insert; lanes 7 and 8: extracts from E. coli AR58 transformed with the plasmid derived from pEXB which expresses P14 cDNA as fusion protein; lane 9: birch pollen protein extract (positive control).

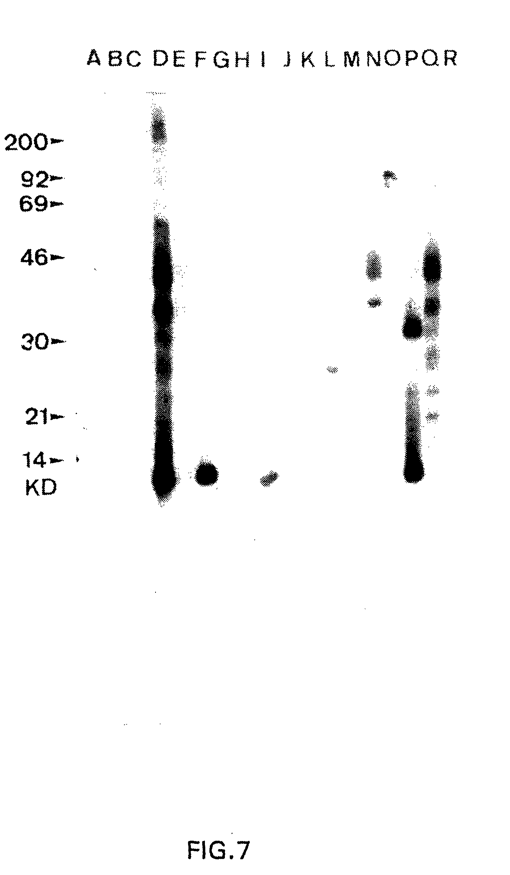

FIG. 7: Sera from various allergic patients were tested for their IgE reactivity with respect to recombinant P14 which was expressed in pKK223-3. Patients (lanes) D, E, F, I, J, and P show IgE binding to the recombinant P14. Lane R is a serum pool from non-allergic individuals. The recombinant P14 was not purified and, therefore, reactivity of patients' IgE with proteins from E. coli was seen.

FIG. 8: Coomassie stained polyacrylamide gel. Lane M: molecular weight marker; lane 1: total pollen proteins of birch; lane 2: birch pollen proteins from which P14 was removed by the affinity method (flow through); lanes 3, 4 and 5: eluted P14. Proteins were applied to the gel and stained to indicate migration. As can be seen from lanes 3, 4 and 5, P14 can be purified by affinity chromatography to poly(L-proline) sepharose.

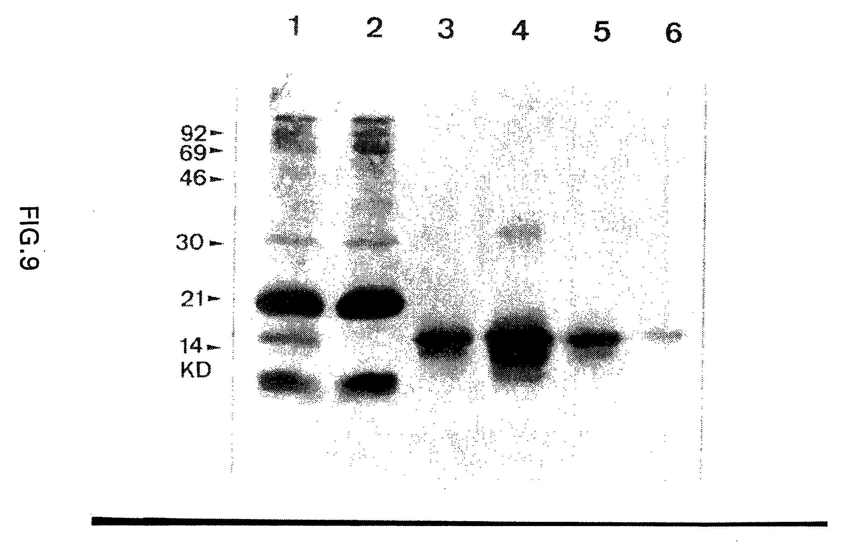

FIG. 9: IgE immunoblot: A probe of proteins obtained in the same way as in FIG. 8 was transferred to nitrocellulose and incubated with serum IgE of a patient who recognizes most birch pollen allergens. Lanes 1 - 5 contain the same material as in FIG. 8; lane 6 contains the molecular weight marker. This immunoblot shows that birch profilin can be purified to apparent homogeneity from other allergens.

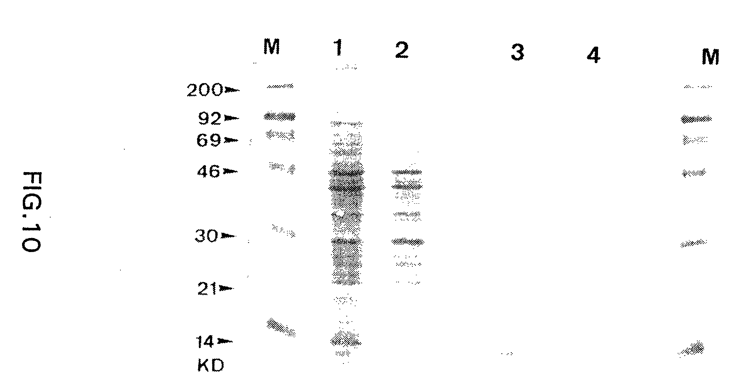

FIG. 10: Coomassie-stained polyacrylamide gel. Lane M: molecular weight marker; lane 1: total proteins of E. coli JM105 with the plasmid derived from pKK223-3 that expressed the P14 cDNA; lane 2: protein fraction after removal of the recombinant P14 by affinity chromatography to poly(L-proline) sepharose; lanes 3 and 4: purified recombinant P14-eluted fractions.

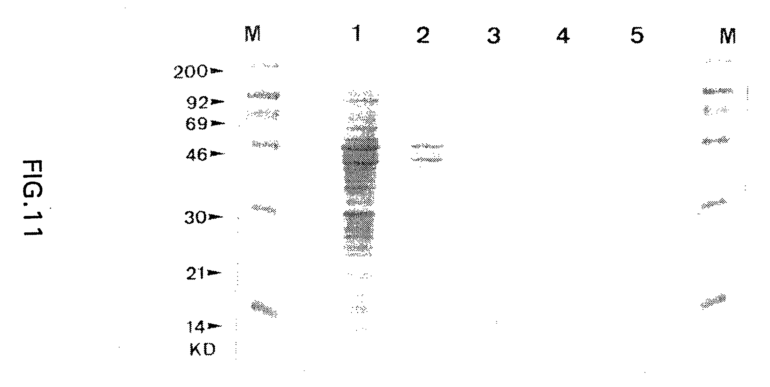

FIG. 11: Coomassie-stained polyacrylamide gel. Lane M: molecular weight marker; lane 1: total protein from E. coli JM105 with the plasmid pKK223-3 without insert; lane 2: protein fraction after poly(L-proline) purification; lanes 3, 4 and 5: eluted fractions. These results show that no protein with similar properties to P14 can be isolated from the expression system without insert.

FIG. 12: IgE immunoblot: Purified human profilin was loaded on a 12% polyacrylamide gel and blotted on nitrocellulose. Strips of the nitrocellulose were cut and incubated as follows: Strip 1 was incubated with serum IgE of a patient who recognized besides P14 and the major birch pollen allergen, Bet v I, allergens in the molecular range between 30 and 90 kD. Strip 2 was incubated with serum IgE from a patient who recognized only P14 in birch pollen extracts; strip 3 was incubated with serum from a patient whose serum IgE was directed only against Bet v I; strip 4 was incubated with the serum from a patient allergic to mites; strip 5 with serum from a group of nonallergic donors and strip 6 shows the buffer control. IgE binding was detected with a ¹²⁵I-labeled antihuman IgE antiserum of rabbit. Cross-reactivity was shown for strips 1 and 2. This data-demonstrates that serum IgE that reacts with birch pollen also cross-reacts with human profilin.

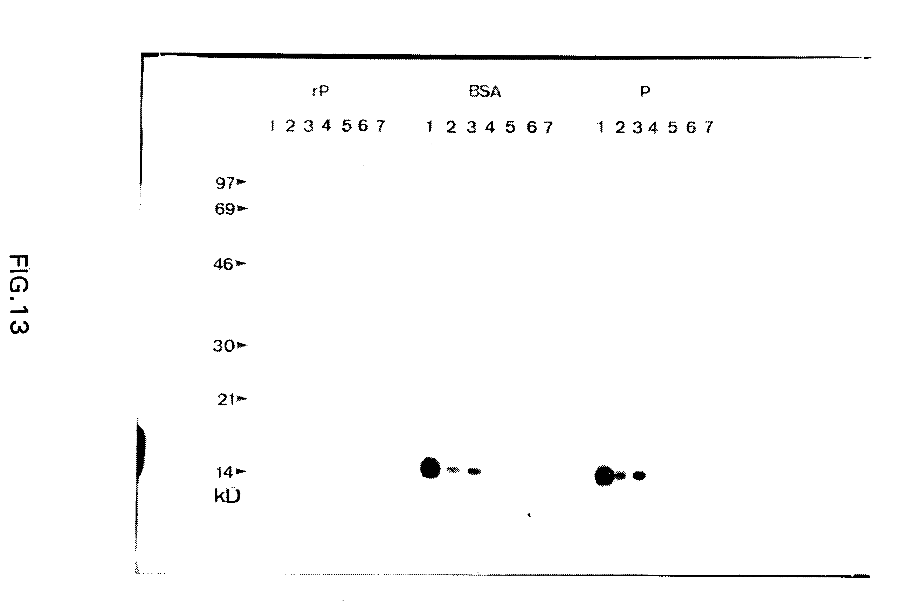

### FIG. 13: IgE-inhibition:

Purified celery profilin was subjected to SDS-Page, blotted to nitrocellulose (1µg/cm). Nitrocellulose strips where incubated with 1:10 dilutions of sera from birch pollen profilin allergic patients (lanes 1-3), from patients allergic to the major birch pollen allergen *Bet ν*I but not to profilin, a serum pool of nonallergic individuals (lane 4) and with buffer without addition of serum (lane 5). The serum dilutions where preincubated with 5µg of purified recombinant birch profilin each (**rP**), 5 µg of BSA (**BSA**), and with serum dilution buffer(**P**). Binding of IgE of the patients 1-3 to celery profilin can be blocked with purified recombinant birch profilin indicating common IgE epitopes of birch and celery profilin.

### FIG. 14: Immunoblots:

Purified celery profilin (C) and recombinant birch profilin (**rP**) is recognized by the rabbit anti celery profilin antibody (R: lane 1). Recombinant birch profilin also binds patients IgE (IgE: lane 1). No binding is seen in lane 2 (buffer control without addition of antibody or serum).

### FIG. 15: Immunoblots:

Purified profilin from rye (*Secale cereale S)* and from mugwort *(Artemisia vulgaris* M) binds the rabbit anti celery profilin antibody (lanes 1) whereas in the buffer control (lane 2) no binding was found.

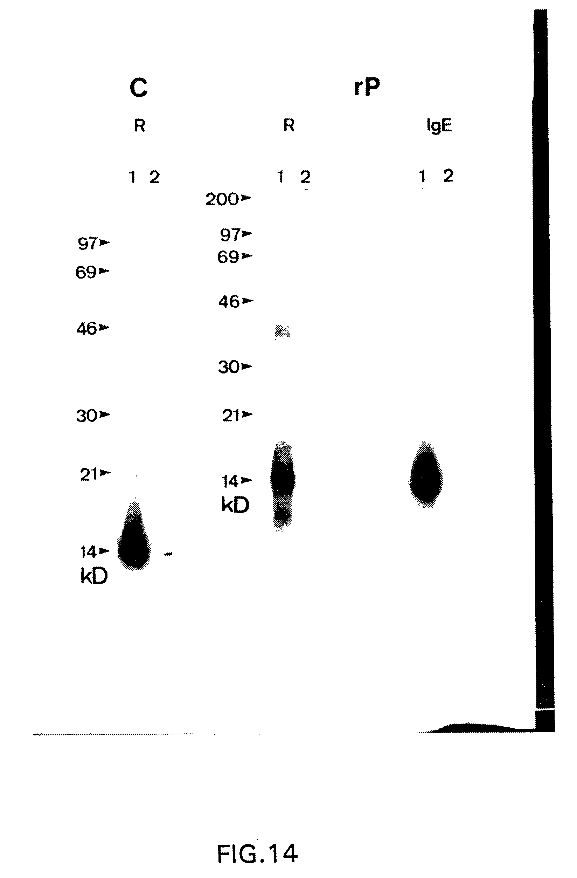

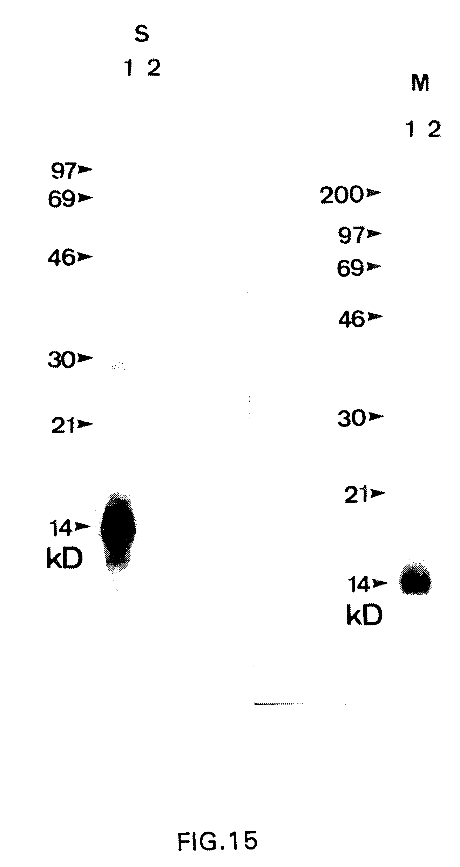

Bound rabbit antibody in Figures 14 and 15 was detected with 125 J donkey anti rabbit antibody from Amerham, UK. Bound serum IgE in Figures 13 and 14 was detected with 125 J rabbit anti human IgE from Pharmacia, Sweden.

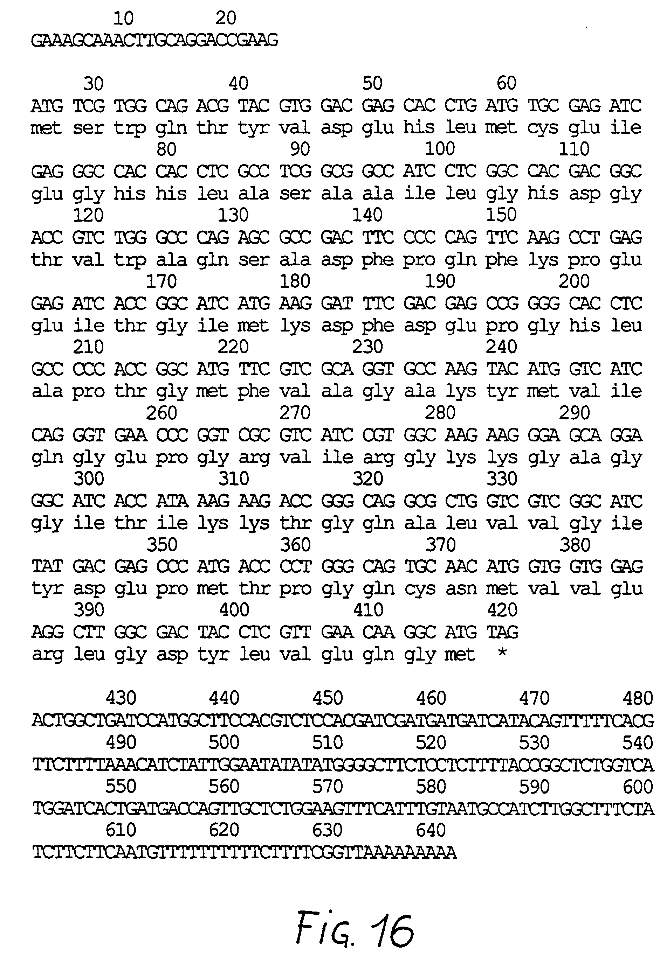

### FIG. 16: cDNA and deduced amino acid sequence of profilin from pollen of timothy grass (Phleum pratense)

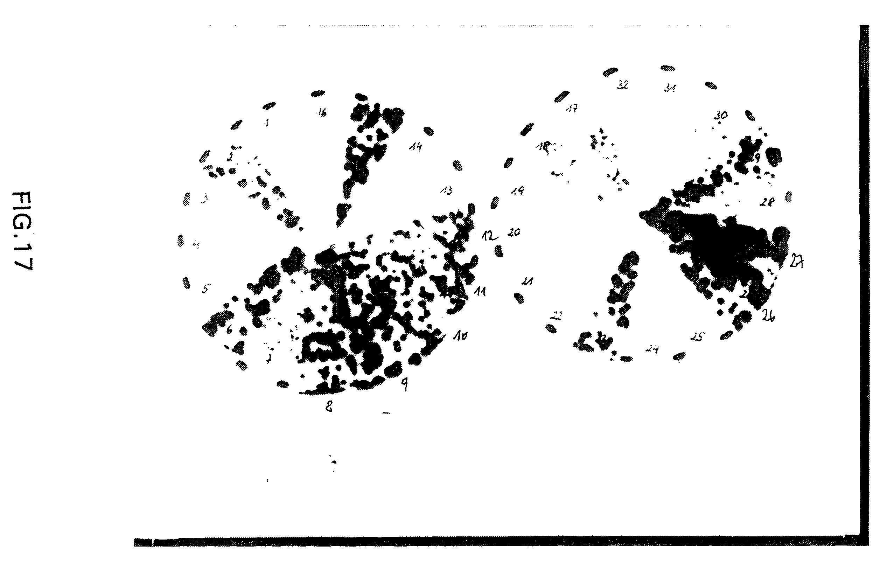

### FIG. 17: IgE-binding of patients IgE to plaquelifts of lambda gt 11 phages containing the cDNA insert encoding timothy grass profilin

30 grass pollen allergic patients were tested for IgE binding to lambda gt 11 phages that express profilin from timothy grass which was bound to nitrocellulose sectors. Sector 31 shows a serumpool from non allergic individuals and sector 32 the buffer control without addition of serum. Serum IgE of patients 2, 6, 7, 8, 9,10,11,12,15,17,18,21,23,26,27,28,29 and 30 bound to the recombinant timothygrass profilin expressed in lambda gt 11. All these patients also displayed IgE reactivity to birch profilin. Patients 1, 3, 4,5,13,14,16,19,20,22,24, and 25 who were allergic to other grass pollen allergens did not show any IgE reactivity to timothy grass profilin. Bound serum IgE was detected as described in Fig. 13.

### 5. EXAMPLES

The invention can be understood by reference to the following examples:

### 5.1. Construction of the cDNA gene bank

Pollen (Allergon AB Engelholm, Sweden) which was examined for purity by means of light and electron microscopy, was used for the isolation of polyadenylated RNA (17, 18). cDNA synthesis was carried out with oligo-dT and random primers (19, 20), the ends of the cDNA were cleanly digested with T4-polymerase and provided with EcoRI-linkers. The cDNA with linkers was ligated and packed in dephosphorylated lambda gtll arms (21). A cDNA gene bank of 800,000 independent clones was produced.

### 5.2. Screening of the cDNA gene bank

IgE screening of the birch pollen cDNA gene bank was performed as described (22). IgE-binding clones were enriched and phage DNA was prepared therefrom (23). The inserts were cut out with EcoRI and the fragments were subcloned in the plasmid pUC18 (24). The DNA sequence of a clone was obtained (25). Although it was complete at the 3'-terminus (poly-A tail), it lacked a part of the 5'-terminus including the start codon as well. This partial sequence is underscored in FIG. 4. Therefore the original gene bank was again screened with oligodeoxynucleotides which were complementary to the coding region (26) and two independent clones were obtained.

### 5.3. RNA (Northern) blots

Ten *µ*g of total RNA from pollen of alder, birch and hazel were separated by means of a denaturing gel electrophoresis and blotted on nitrocellulose (27, 28). A P14 cDNA probe, as underlined in FIG. 4, was ³²P-labeled by means of random priming (29). Prehybridization and hybridization were carried out by standard methods (23). The blots were washed with 0.75xSSC (20xSSC = 3M NaCl, 0.3M Na citrate, pH 7.0), 0.1 % SDS (sodium dodecyl sulfate) at 50°C and autoradiographed (Hyperfilm MP, Amersham, London, UK).

### 5.4. Expression of Birch P14 cDNA

### 5.4.1 Expression of the 3'-terminus of the cDNA in lambda gtll phages (FIG. 2)

An incomplete cDNA clone which codes for a part of P14 was obtained by means of IgE screening (22) as described in Section 5.2. The lysogenic E. coli strain Y1089 was inoculated with recombinant lambda gt11 phages, containing an insert as underlined in FIG. 4, and the β-galactosidase fusion protein was recovered from the mixture (19). The construction would predict that a fusion protein having a molecular weight of 116 kD would be produced. The mixture was subjected to electrophoresis on a 7.5% polyacrylamide gel and was blotted on nitrocellulose. The fusion protein was detected by means of IgE antibodies in patient serum and an iodine-labeled rabbit antihuman IgE antibody (Pharmacia, Uppsala, Sweden) (FIG. 2). As shown in FIG. 2, a fusion protein having a molecular weight of between 115 kD and capable of binding to IgE antibodies was observed.

### 5.4.2. Expression of complete P14 cDNA as fusion and nonfusion protein

The complete cDNA that codes for P14 contains a prokaryotic ribosome binding site (Shine-Dalgarno sequence (30)) and was inserted into the EcoRI sites of plasmids pKK223-3 (31) or pEXB (32) to obtain P14 as a nonfusion protein or a fusion protein of P14 with the lambda cII protein. IgE-binding clones were obtained by means of serum IgE and a colony screening method (33) and were examined by means of DNA restriction analysis. Recombinant proteins were tested for their binding capacity with respect to patient IgE antibodies as described (22) (FIG. 6).

### 5.5. Purification of birch pollen P14 and recombinant P14

P14 from birch pollen and recombinant P14 were purified by means of an affinity method by a batch process (cf. 15, 16) which is suitable for the profilins of Acanthamoeba (10), yeast (11) and man (13). Birch pollen and E. coli cells, which contain the plasmid that codes for P14, were lysed in PHEM-TX buffer (2x PHEM-TX: 120 mM PIPES (piperazine-1,4-bis(2-ethanesulfonic acid)), 50 mM HEPES (N-2-hydroxyethylpiperazine-N'-2-ethanesulfonic acid), 20 mM EGTA (ethylene glycol-bis(β-aminoethyl ether)-N,N,N',N'-tetraacetic acid), 4 mM MgCl₂, 10 mM glucose, 20 *µ*g/ml leupeptin, 156 *µ*g/ml benzamidin, 80 *µ*g/ml aprotinin, 1 mM PMSF (phenylmethyl sulfonyl fluoride), 1.5% Triton-X100, pH 7.2) and the lysate was centrifuged for one hour at 65000 x g at 4°C. The supernatant was incubated overnight at 4°C with poly(L-proline) coupled to BrCN-activated Sepharose 4B (Pharmacia, Uppsala, Sweden). Then the affinity matrix was washed three times with a double volume each time of TBS-ATP (20 mM TRIS, 150 mM NaCl, 0.5 mM ATP (adenosine triphosphate), pH 7.6) and then eluted for five minutes at room temperature with a double volume of elution buffer I (TBS-ATP with 2M urea). The supernatant was collected. The procedure was repeated twice with elution buffer II (TBS-ATP with 6M urea) and the supernatants were dialyzed against distilled water at 4°C. The dialysates, which contained the proteins, were lyophilized and analyzed by means of polyacrylamide gel electrophoresis and IgE immunoblot (FIGS. 8, 9, 10 and 11).

### 5.6. IgE-binding capacity of a protein expressed from a fragment of P14 cDNA which contains an IgE-binding epitope

The 3'-region of P14 cDNA (bp 419-478) was cloned in the EcoRI site of lambda gtll and expressed as an IgE-binding polypeptide (21) as shown in FIG. 2. The β-galactosidase fusion protein (lane 4) bound strongly to IgE of the patient, while the control lanes 1 and 2 exhibited no IgE binding for the proteins of E. coli Y1089 and the proteins of E. coli Y1089 which were inoculated with lambda gtll phages without an insert. This example shows that a partial cDNA clone which codes for a protein having at least one epitope of the P14 molecule was obtained. It follows from this that partial cDNA clones which code for such incomplete P14 polypeptides may be useful for a therapy or diagnosis in a way similar to the complete P14 molecule or homologous proteins.

### 5.7. Demonstration of polynucleotides and polypeptides homologous to P14 within the order Fagales

The Northern (RNA) blot (FIG. 3) shows that the P14 cDNA sequence (polynucleotide underlined in FIG. 4) is able to cross-hybridize with pollen mRNA from alder and hazel under stringent conditions (requirements of stringency are defined in 3. Summary of the Invention). Therefore, the sequence homology of the corresponding allergens of trees of the order Fagales can already be demonstrated at the nucleic acid level. FIG. 1A already showed a similar IgE-binding capacity of proteins of alder, hazel and hornbeam homologous to P14. It follows from this that P14 cDNA codes for polypeptides of similar IgE-binding capacity and antigenicity to closely related tree pollen allergens.

### 5.8. Seguence analysis

FIG. 4 shows the sequence of the cDNA that codes for birch P14, and the deduced amino acid sequence of the coding region. It contains the complete protein coding region. The sequence of the peptide that, coupled to β-galactosidase, represents an IgE-binding epitope is underlined in the figure (see Example 5.4).

FIG. 5 illustrates the sequence homology between the P14 protein of birch and of human, mouse, calf, yeast and Acanthamoeba profilins (13, 12, 14, 11, 10).

The cross-reactivity of patient IgE with birch P14 and human profilin is shown in FIG. 12. Similar chemical properties of these related proteins were likewise shown by their common affinity to poly(L-proline) (FIGS. 8 and 10). These data indicate that the profilins of species which are as far apart in evolutionary terms as humans and birch are able to act as cross-reactive panallergens that may lead to an IgE autoimmune reactivity in patients.

### 5.9. Expression of P14 coding cDNA in E. coli as fusion or nonfusion protein and detection of IgE-binding capacity of these polypeptides

The polynucleotide given in FIG. 4 (nucleotides 1-710) that codes for birch P14 was inserted in the plasmid pKK223-3 so that a recombinant nonfusion protein (31) could be prepared, while a recombinant fusion protein was produced in the plasmid pEXB (32). The reactivity of these polypeptides with patient IgE is shown in FIG. 6. Control protein extracts of E. coli in lanes 1, 2, 5 and 6 do not bind IgE, while recombinant birch P14 expressed as a nonfusion protein (lanes 3 and 4) and as a fusion protein (lanes 7 and 8) does bind IgE.

In FIG. 7, sera from persons allergic to birch pollen (A-K), to grass pollen (L-N) and to mugwort (O-Q) and of a pool of nonallergic individuals (R), all of whom had been selected according to their case history, RAST and skin test, were tested for their IgE-binding capacity with recombinant birch P14. IgEs of Sera D, E, F, I, J and P bound to P14 expressed in pKK223-3.

It follows from this that this invention provides a polynucleotide that codes for polypeptides which have similar antigenicity and similar IgE-binding capacity to the P14 protein of birch when the polynucleotide is inserted in the correct reading frame of a variety of expression systems. The IgE-binding properties of these polypeptides were demonstrated for sera from patients who exhibit allergic reactions to various pollens and hence point to the great clinical significance of these polypeptides (FIG. 7).

### 5.10. Purification of P14 from pollen and of recombinant P14 from E. coli

As described above, this invention provides a simple method for purifying natural as well as recombinant P14. The Coomassie-stained polyacrylamide gel in FIG. 8 shows that pure P14 (lanes 3, 4 and 5) can be separated from total pollen protein (lane 1). The proteins which do not bind to poly(L-proline)-Sepharose are also shown (lane 2). The effectiveness of this purification method was monitored by means of IgE immunoblotting (FIG. 9), and for this purpose serum from a patient who recognizes most birch pollen allergens with IgE antibodies (lane 1) was used. After application of the affinity method, almost no P14 can be found (lane 2), while purified P14 was obtained in lanes 3, 4 and 5.

FIG. 10 shows a polyacrylamide gel which demonstrates the purification of recombinant P14 from E. coli JM105 that is transformed with the plasmid pKK223-3, which carries the P14 coding sequence. Recombinant P14 (lanes 3 and 4) was purified from the total proteins by affinity chromatography to poly(L-proline) sepharose (lane 1), the remaining proteins being shown by lane 2. FIG. 11 shows that no homologous protein from E. coli JM105 transformed with pKK223-3 without insert is obtained by means of the method used.

As FIGS. 9, 6 and 7 show, the purified protein (from birch and E. coli) retains its IgE-binding capacity. This example thus shows that the present invention likewise provides a simple and rapid purification method for P14 both as natural and recombinant polypeptide. Using poly(L-proline) to purify, both natural and recombinant P14 retain their antigenicity and IgE binding capacity. In addition, the method offers the opportunity to immobilize (and separate by affinity means) the immunologically active polypeptide.

### 5.11. IgE reactivity of allergic and atopic patients with human profilin

Various patient sera were selected as follows (FIG. 12): Patient 1 shows IgE antibodies which are directed against most birch pollen allergens, including BetvI and P14, patient 2 shows IgE binding only with P14 and patient 3 only with BetvI. Patient 4 is a person allergic to house dust mite and the serum pool 5 was made up of nonallergic individuals. Strip 6 is the buffer control. All these sera were tested for their IgE-binding capacity with nonrecombinant and recombinant P14 and human profilin. Those patients (FIG. 12), who recognized the nonrecombinant P14 from birch as well as the recombinant P14 from E. coli, also had IgE antibodies against human profilin. For this reason, this invention for the first time gives indications on the molecular level that autoimmune mechanisms might play a role in atopic and allergic diseases. Since patients with other autoimmune diseases form antibodies against P14, this invention should provide a diagnostic marker for these diseases.

### 5.12. Correlation of case histories of atopic and allergic patients with the binding of IgE antibodies to P14

The case histories of patients who form IgE antibodies against P14 show that all of them suffer from severe allergic symptoms which are caused by a great variety of allergens (tree and grass pollen, mite, cat and dog allergens), that they have an elevated total IgE level and show an unsatisfactory course in hyposensitization therapy. It follows from this that a positive reaction of the serum IgE of patients with P14 is usable as a good marker for the differentiation of certain groups of atopic and allergic patients.

### 5. 13. Demonstration of common IgE-epitopes between profilins in food (celery) and pollen P14 allergen (birch)

The IgE-inhibition experiment shown in Figure 13 shows that there is common IgE-binding capacity of proteins homologous to the P14 allergen in pollens and food. Purified recombinant birch profilin, when added to the patients' serum in the fluid phase before the serum is incubated with the nitrocellulose bound celery profilin, is able to completely block the binding of patients IgE to celery profilin. This indicates that the P14 allergen of birch (birch profilin) contains all IgE epitopes that can be found in celery profilin. Recombinant birch P14 allergen is therefore suitable not only for diagnosis and therapy of pollen allergies but also for food allergies. Common antigenicity of birch pollen P14 allergen and food profilins is also shown by crossreactivity of a rabbit anti celery profilin antibody with the pollen P14 allergen (Figs. 14 and 15).

### 5. 14. Sequence similarity and common IgE-binding capacity of pollen P14 allergen from white birch (Betula verrucosa) and the homologous P14(T) allergen from timothy grass (Phleum pratense).

Amino acid sequence identity of birch P14 allergen and timothy grass P14(T) allergen is 77%. The cDNA encoding the P14(T) allergen from timothy grass was obtained by screening a cDNA library, which was constructed from pollen of timothy grass in the same way as described for the cDNA library from birch pollen, with patients' IgE as described for the P14 allergen of birch. The cDNA sequence of the clone encoding the P14(T) allergen of timothy grass is shown in Fig. 16. and as sequences 9-11 in the sequence listing. All patients' sera that bound with their IgE to birch profilin also bound with their IgE to sectors of nitrocellulose filters containing plaquelifts of immunopositive lambda gt11 phages into which the cDNA encoding timothy grass P14(T) allergen had been inserted (Fig. 17). This shows that the proteins related to the P14 allergens by high homology also are immunologically cross reactive with patients' IgE antibodies.

### 6. METHODS OF ADMINISTRATION

The present invention covers the use of P14 synthetic polypeptide allergens to hyposensitize or desensitize a mammal. Such polypeptides can be administered to a human subject either alone or in combination with pharmaceutically acceptable carriers or diluents, in accordance with standard pharmaceutical practice.

The method of hyposensitization involves or could involve the successive parenteral oral, nasal, inhalant or rectal administration of incremental doses of the P14 allergen. The term parenteral as used herein includes subcutaneous, intravenous or intramuscular injections.

A range from 1 picogram to 10 milligrams per application can be used. The diluents and carriers can be chosen by those skilled in the art according to commonly accepted galenic procedures.

### 7. REFERENCES

The references cited in the above specification are:
1. L. Yman, Botanical relations and immunological cross-reactions in pollen allergy, 2nd ed. Uppsala, Sweden: Pharmacia AB, 1982.
2. W. R. Thomas, K. Y. Chua, W. K. Greene and G. A. Stewart. Recombinant mite allergens. In: Epitopes of atopic allergens. A. H. Sehon, D. Kraft, and G. Kunkel (eds). UCB Institute of Allergy, Brussels 1990.
3. E. Jarolim, M. Tejkl, M. Rohac, G. Schlerka, M. Breitenbach, O. Scheiner, D. Kraft, H. Rumpold. Monoclonal antibodies against birch pollen allergens; characterization by immunoblotting and use for single step affinity purification of the major allergen BetvI. Int. Arch. Allergy Appl. Immunol. 90, 54-60 (1989).
4. H. Ipsen, H. Bowadt, H. Janniche, B. Nüchel Petersen, E. P. Munch, J. A. Wihl and H. Lowenstein. Immunochemical characterization of reference alder (Alnus glutinosa) and hazel (Corylus avellana) pollen extracts and the partial immunochemical identity between the major allergens of alder, birch, and hazel pollen. Allergy 40, 510-518 (1985).
5. H. Rumpold, M. Rohac, B. Bohle, M. Breitenbach, O. Scheiner and D. Kraft. The relationship of *Betv*I epitopes recognized by patients' IgE and monoclonal anti-*Betv*I antibodies. In: Epitopes of atopic allergens. A. Sehon, D. Kraft and G. Kunkel (eds). The UCB Institute of Allergy, Brussels 1990.
6. R. Valenta, H. Breiteneder, K. Pettenburger, M. Breitenbach, H. Rumpold, D. Kraft and O. Scheiner. Homology of the major pollen allergens of alder, hazel, and hornbeam at the nucleic acid level as determined by cross-hybridization. J. Allergy Clin. Immunol., in press.
7. B. Nüchel Petersen, H. Janniche, E.P. Munch, J. A. Wihl, H. Böwadt, H. Ipsen and H. Lowenstein. Immunotherapy with partially purified and standardized tree pollen extracts. Allergy 43, 353-362 (1988).
8. J. A. Wihl, H. Ipsen, B. Nüchel Petersen, E. P. Munch, H. Janniche and H. Lowenstein. Immunotherapy with partially purified tree pollen extracts. Allergy 43, 363-369 (1988).
9. H. Ipsen, B. Schwartz, J. A. Wihl, B. Nüchel Petersen, E. P. Munch, H. Janniche and H. Lowenstein. Immunotherapy with partially purified and standardized tree pollen extracts. Allergy 43, 370-377 (1988).
10. C. Ampe, J. Vandekerckhove, S. L. Brenner, L. Tobacman and E. D. Korn. The amino acid sequence of Acanthamoeba profilin. J. Biol. Chem. 260, 834-840 (1985).
11. V. Magdolen, U. Oechsner, G. Holler and W. Bandlow. The intron-containing gene for yeast profilin (PFY) encodes a vital function. Mol. Cell. Biol. 8, 5108-5115 (1988).
12. J. S. Widada, C. Ferraz and J.-P. Liautard. Total coding sequence of profilin cDNA from *Mus musculus* macrophage. Nucl. Acids Res. 17, 2855 (1989).
13. D. J. Kwiatkowski and G. A. P. Bruns. Human profilin. Molecular cloning, sequence comparison, and chromosomal analysis. J. Biol. Chem. 263, 5910-5915 (1988).
14. C. Ampe, F. Markey, U. Lindberg and J. Vandekerckhove. The primary structure of human platelet profilin: reinvestigation of the calf spleen profilin sequence. FEBS Lett. 228, 17-21 (1988).
15. Lindberg, C. E. Schutt, E. Hellsten, A.-C. Tjäder und T. Hult. The use of poly(L-proline)-Sepharose in the isolation of profilin and profilactin complexes. Biochim. Biophys. Acta 967, 391-400 (1988).
16. M. Tanaka and H. Shibata. Poly(L-proline)-binding proteins from chick embryos are a profilin and a profilactin. Eur. J. Biochem. 151, 291-297 (1985).
17. H. Breiteneder, W. Hassfeld, K. Pettenburger, E. Jarolim, M. Breitenbach, H. Rumpold, D. Kraft and O. Scheiner. Isolation and characterization of messenger RNA from male inflorescences and pollen of white birch (*Betula verrucosa).* Int. Arch. Allergy Appl. Immunol. 87, 19-24 (1988).
18. H. Aviv und P. Leder. Purification of biologically active globin messenger RNA by chromatography on oligothymidylic acid-cellulose. Proc. Natl. Acad. Sci. USA 69, 1408-1412 (1972).
19. T. V. Huynh, R. A. Young, R. W. Davis, In: DNA cloning - a practical approach, Band 1, D. M. Glover (ed), IRL Press, Oxford 1985.
20. H. Haymerle. Nucl. Acids Res. 14, 8615 (1986).
21. R. A. Young and R. W. Davis. Efficient isolation of genes by using antibody probes. Proc. Natl. Acad. Sci. USA 80, 1184-1198 (1983).
22. H. Breiteneder, K. Pettenburger, A. Bito, R. Valenta, D. Kraft, H. Rumpold, O. Scheiner and M. Breitenbach. The gene coding for the major birch pollen allergen *Betv*I is highly homologous to a pea disease resistance response gene. EMBO J. 8, 1935-1938 (1989).
23. F. M. Ausubel. Current protocols in molecular biology. Green Publishing Associates and Wiley-Interscience, New York, 1987.
24. C. Yanisch-Perron, J. Vieira and J. Messing. Improved M13 phage cloning vectors and host strains: nucleotide sequences of the M13mp18 and pUC19 vectors. Gene 33, 103-119 (1985).
25. F. Sanger, S. Nicklen and A. R. Coulson. DNA sequencing with chain-terminating inhibitors. Proc. Natl. Acad. Sci. USA 74, 5463-5467 (1977).
26. R. B. Wallace, J. Shaffer, R. F. Murphy, J. Bonner, T. Hirose and K. Itakura. Hybridization of synthetic oligodeoxyribonucleotides to ΦX 174 DNA: the effect of a single base pair mismatch. Nucleic Acids Res. 6, 3543-3557 (1979).
27. P. S. Thomas. Hybridization of denatured RNA and small DNA fragments transferred to nitrocellulose. Proc. Natl. Acad. Sci. USA 77, 5201-5205 (1980).
28. H. Lehrach. RNA molecular weight determinations by gel electrophoresis under denaturing conditions: a critical reexamination. Biochemistry 16, 4743-4751 (1977).
29. A. P. Feinberg and B. Vogelstein. A technique for radiolabeling DNA restriction fragments to high specific activity. Anal. Biochem. 132, 6-13 (1983).
30. J. Shine and L. Dalgarno. The 3'-terminal sequence of Escherichia coli 16S ribosomal RNA: complementarity to nonsense triplets and ribosome binding sites. Proc. Natl. Acad. Sci. USA 71, 1342-1346 (1974).
31. E. Amann, J. Brosius and M. Ptashne. Vectors bearing a hybrid trp-lac promoter useful for regulated expression of cloned genes in Escherichia coli. Gene 25, 167-178 (1983).
32. N. Kiyoshi, H.C. Thøgersen. Generation of β-globin by sequence-specific proteolysis of a hybrid protein produced in Escherichia coli. Nature 309, 810-812 (1984).
33. D.M. Helfman, J.R. Feramisco, J.C. Fiddes, G.P. Thomas and S.H. Hughes. Identification of clones that encode chicken tropomyosin by direct immunological screening of a cDNA expression library. Proc. Natl. Acad. Sci. USA 80, 31-35 (1983).

## Claims

1. A recombinant DNA molecule consisting of the sequence of SEQ ID NO:2.

2. A replicable microbial expression vehicle which directs expression of a DNA of claim 1 to produce said protein.

3. A prokaryotic host cell containing a DNA according to claim 1, wherein said DNA replicates and expresses the P14 allergen.

4. A recombinant DNA molecule encoding a protein consisting SEQ ID NO:3.

5. A recombinant DNA molecule consisting of sequence of SEQ ID NO: 1.

6. A recombinant DNA molecule, comprising DNA that is complementary to a nucleic acid molecule that hybridizes under stringent conditions to a DNA molecule consisting of the nucleic acid sequence of SEQ ID NO:2 and which encodes a protein that (a) binds to IgE antibodies in serum of an individual allergic to an allergen consisting of the amino acid sequence of SEQ ID NO:3 and (b) binds to poly-(L-proline), wherein said stringent conditions are: at 55°C, a salt concentration of 150 mM NaCl and 15 mM Na₃citrate x 2H₂O, at pH 7,0 and with a sodium dodecyl sulfate at a concentration of 0,1 % weight/volume.

7. A recombinant DNA molecule according to claim 6, wherein the protein is a P14 allergen selected from the group consisting of birch, alder, hazel, hornbeam and oak.

8. A recombinant DNA molecule according to claim 6, wherein the protein is a P14 allergen of birch.

9. A recombinant DNA molecule according to any one of claims 6, 7 or 8 which codes for a protein consisting of the amino acid sequence of SEQ ID NO:3.

10. A replicable microbial expression vehicle which directs expression of a DNA of any one of claims 6, 7 or 8 to produce said protein.

11. A replicable microbial expression vehicle which directs expression of a DNA of claim 9 to produce said protein.

12. A prokaryotic or eukaryotic host cell containing a microbial expression vehicle which directs expression of a DNA of any one of claims 6, 7 or 8 to produce said protein.

13. A prokaryotic or eukaryotic host cell containing a microbial expression vehicle which directs expression of a DNA of claim 9 to produce said protein.

14. A host organism according to claim 12, wherein the host cell is *Escherichia coli*.

15. A host organism according to claim 13, wherein the host cell is *Escherichia coli.*

## Patentansprüche

1. Rekombinantes DNA-Molekül, bestehend aus der Sequenz SEQ ID NR: 2.

2. Replizierbares, mikrobielles Expressionsvehikel, welches die Expression einer DNA nach Anspruch 1 zur Produktion des Proteins lenkt.

3. Prokaryotische Wirtszelle, enthaltend eine DNA nach Anspruch 1, wobei die DNA repliziert und das P14 Allergen exprimiert.

4. Rekombinantes DNA-Molekül, das für ein Protein kodiert, welches aus der Sequenz SEQ ID NR:3 besteht.

5. Rekombinantes DNA-Molekül, bestehend aus der Sequenz SEQ ID NR: 1.

6. Rekombinantes DNA-Molekül, umfassend DNA, die zu einem Nukleinsäuremolekül komplementär ist, das unter stringenten Bedingungen an ein DNA-Molekül hybridisiert, das aus der Nukleinsäuresequenz SEQ ID NR:2 besteht, und ein Protein kodiert, das (a) an IgE-Antikörper in Serum eines Individuums bindet, das auf ein Allergen allergisch ist, welches aus der Aminosäuresequenz SEQ ID NR.3 besteht, und (b) an Poly-(L-Prolin) bindet, wobei die stringenten Bedingungen sind: bei 55 °C, eine Salzkonzentration von 150mM NaCl und 15 mM Na₃citrat x 2H₂O, bei pH 7,0 und mit einem Natriumdodecylsulfat bei einer Konzentration von 0,1 % Gewicht/Volumen.

7. Rekombinantes DNA-Molekül nach Anspruch 6, wobei das Protein ein P14-Allergen ist, ausgewählt aus der Gruppe bestehend aus, Birke, Erle, Hasel, Hain- bzw. Weißbuche und Eiche.

8. Rekombinantes DNA-Molekül nach Anspruch 6, wobei das Protein ein P14-Allergen der Birke ist.

9. Rekombinantes DNA-Molekül nach einem der Ansprüche 6, 7 oder 8, das für ein Protein kodiert, das aus der Aminosäuresequenz SEQ ID NR:3 besteht.

10. Replizierbares, mikrobielles Expressionsvehikel, welches die Expression einer DNA nach einem der Ansprüche 6, 7 oder 8 zur Produktion des Proteins lenkt.

11. Replizierbares, mikrobielles Expressionsvehikel, welches die Expression einer DNA nach Anspruch 9 zur Produktion des Proteins lenkt.

12. Prokaryotische oder eukaryotische Wirtszelle, die ein replizierbares, mikrobielles Expressionsvehikel enthält, welches die Expression einer DNA nach einem der Ansprüche 6, 7 oder 8 zur Produktion des Proteins lenkt.

13. Prokaryotische oder eukaryotische Wirtszelle, die ein replizierbares, mikrobielles Expressionsvehikel enthält, welches die Expression einer DNA nach Anspruch 9 zur Produktion des Proteins lenkt.

14. Wirtsorganismus nach Anspruch 12, wobei die Wirtszelle *Escherichia coli* ist.

15. Wirtsorganismus nach Anspruch 13, wobei die Wirtszelle *Escherichia coli* ist.

## Revendications

1. Molécule d'ADN recombinant constituée de la séquence de SEQ ID NO:2.

2. Vecteur d'expression microbien capable de réplication qui dirige l'expression d'un ADN de la revendication 1 pour produire ladite protéine.

3. Cellule hôte procaryote contenant un ADN selon la revendication 1, dans laquelle ledit ADN se réplique et exprime l'allergène P14.

4. Molécule d'ADN recombinant codant pour une protéine constituant SEQ ID NO:3.

5. Molécule d'ADN recombinant constituée de la séquence de SEQ ID NO:1.

6. Molécule d'ADN recombinant, comprenant un ADN qui est complémentaire d'une molécule d'acide nucléique qui s'hybride dans des conditions stringentes à une molécule d'ADN constituée de la séquence d'acide nucléique de SEQ ID NO:2 et qui code pour une protéine qui (a) se lie aux anticorps IgE dans le sérum d'un individu allergique à un allergène constitué de la séquence d'acides aminés de SEQ ID NO:3 et (b) se lie à une poly-(L-proline), où lesdites conditions stringentes sont : à 55°C, une concentration en sel de 150 mM de NaCl et 15 mM de Na₃citrate x 2H₂O, à pH 7,0 et avec du dodécylsulfate de sodium à une concentration de 0,1% poids/volume.

7. Molécule d'ADN recombinant selon la revendication 6, dans laquelle la protéine est un allergène P14 choisi dans le groupe constitué du bouleau, de l'aulne, du coudrier, du charme et du chêne.

8. Molécule d'ADN recombinant selon la revendication 6, dans laquelle la protéine est un allergène P14 de bouleau.

9. Molécule d'ADN recombinant selon l'une quelconque des revendications 6, 7 ou 8 qui code pour une protéine constituée de la séquence d'acides aminés de SEQ ID NO:3.

10. Vecteur d'expression microbien capable de réplication qui dirige l'expression d'un ADN de l'une quelconque des revendications 6, 7 ou 8 pour produire ladite protéine.

11. Vecteur d'expression microbien capable de réplication qui dirige l'expression d'un ADN de la revendication 9 pour produire ladite protéine.

12. Cellule hôte procaryote ou eucaryote contenant un vecteur d'expression microbien qui dirige l'expression d'un ADN de l'une quelconque des revendications 6, 7 ou 8 pour produire ladite protéine.

13. Cellule hôte procaryote ou eucaryote contenant un vecteur d'expression microbien qui dirige l'expression d'un ADN de la revendication 9 pour produire ladite protéine.

14. Organisme hôte selon la revendication 12, dans lequel la cellule hôte est *Escherichia coli*.

15. Organisme hôte selon la revendication 13, dans lequel la cellule hôte est *Escherichia coli*.


### 8. SEQUENCE LISTING

### (1) GENERAL INFORMATION

(i) APPLICANT:
   Dr. Rudolf Valenta
   Dr. Michael Duchene
   Mrs. Dr. Karin Pettenburger
   Dr. Michael Breitenbach
   Dr. Dietrich Kraft
   Dr. Helmut Rumpold
   Dr. Otto Scheiner
(ii) TITLE OF INVENTION: Birch Pollen Allergen P14 for Diagnosis and Therapy of Allergic Diseases
(iii) NUMBER OF SEQUENCES: 8
(iv) CORRESPONDENCE ADDRESS:
   (A) ADDRESSEE: Pennie & Edmonds
   (B) STREET: 1155 Avenue of the Americas
   (C) CITY: New York
   (D) STATE: New York
   (E) COUNTRY: USA
   (F) ZIP: 10036
(v) COMPUTER READABLE FORM:
   (A) MEDIUM TYPE: Diskette, 3.50 inch
   (B) COMPUTER: Hewlett Packard (IBM-PC Compatible)
   (C) OPERATING SYSTEM: MS-DOS
   (D) SOFTWARE: WordPerfect 5.1
(vi) CURRENT APPLICATION DATA:
   (A) APPLICATION NUMBER: 8
   (B) FILING DATE:
   (C) CLASSIFICATION:
(vii) PRIOR APPLICATION DATE:
   (A) APPLICATION NUMBER: 07/353,844
   (B) FILING DATE: May 18, 1989
(viii) ATTORNEY/AGENT INFORMATION:
   (A) NAME: Harry C. Jones, III
   (B) REGISTRATION NUMBER: 20,280
   (C) REFERENCE/DOCKET NUMBER: 6530-008
(ix) TELECOMMUNICATION INFORMATION:
   (A) TELEPHONE: (212) 790-9090
   (B) TELEFAX: (212) 869-9741/8864

### (2) INFORMATION FOR SEQ ID NO:1:

(i) SEQUENCE CHARACTERISTICS:
   (A) LENGTH: 700 nucleotides
   (B) TYPE: nucleic acid
   (C) STRANDEDNESS: single
   (D) TOPOLOGY: linear
(ii) MOLECULE TYPE: cDNA of mRNA
(iii) HYPOTHETICAL: No
(iv) ANTI-SENSE: No
(v) FRAGMENT TYPE: Not Applicable
(vi) ORIGINAL SOURCE:
   (A) ORGANISM: Betula verrucosa
(vii) IMMEDIATE SOURCE:
   (A) POLLEN FROM ALLERGON AB, ENGELHOLM, SWEDEN
(viii) POSITION IN GENOME: Not applicable
(ix) FEATURE: Not Applicable
(x) PUBLICATION INFORMATION: Not Applicable
(xi) SEQUENCE DESCRIPTION: SEQ ID NO:1:
   CAGAGAAAGC GAAAGCTCTC CGCCACAACA AAACGAAGTA GAAGAAGAAG AGTGAGCAAG  60
   AGACAGAGGG AAGAGGAAAA TGTCGTGGCA AACGTACGTG GATGAACATT TGATGTGCGA 120
   TATCGACGGG CAAGCCAGCA ACTCGCTGGC ATCTGCGATC GTCGGTCACG ATGGCTCTGT 180
   GTGGGCCCAG AGCTCTTCCT TCCCACAGTT TAAGCCTCAG GAAATCACTG GTATCATGAA 240
   GGACTTTGAG GAGCCGGGTC ATCTTGCTCC GACGGGCTTA CACCTTGGGG GCATAAAATA 300
   CATGGTCATC CAGGGAGAGG CTGGTGCTGT CATCCGTGGA AAGAAGGGAT CTGGAGGTAT 360
   TACTATAAAG AAGACTGGTC AAGCTCTCGT TTTTGGCATC TATGAAGAGC CTGTGACACC 420
   AGGACAGTGC AACATGGTTG TTGAGAGGTT GGGGGATTAC CTTATTGACC AGGGCCTGTA 480
   GGCAAAGGTC TATCATCATT TGGGGCTTAA TTGTTTTTTT TTTTTTTTTG CTCTTATTCC 540
   CTTTGATTTC GGTTCCAAGT GTGCATCGAT CTTCATTTGA AAGCCTTAAA TTGGCAGTGA 600
   AGTTGTTGCA GACAATAACC ATGTGAGAAC TAAAACATTT GTCTTGTGTT TGGTTGTTTG 660
   AAAAAAAAAA AAAAAAAAAA AAAAAAAAAA AAAAAAAAAA                       700

### (3) INFORMATION FOR SEQ ID NO:2:

(i) SEQUENCE CHARACTERISTICS:
   (A) LENGTH: 399 nucleotides
   (B) TYPE: nucleic acid
   (C) STRANDEDNESS: single
   (D) TOPOLOGY: liner
(ii) MOLECULE TYPE: cDNA of mRNA
(iii) HYPOTHETICAL: No
(iv) ANTI-SENSE: No
(v) FRAGMENT TYPE: Not Applicable
(vi) ORIGINAL SOURCE:
   (A) ORGANISM: Betula verrucosa
(vii) IMMEDIATE SOURCE:
   (A) POLLEN FROM ALLERGON AB, ENGELHOLM, SWEDEN
(viii) POSITION IN GENOME: Not Applicable
(ix) FEATURE: Not Applicable
(x) PUBLICATION INFORMATION: Not Applicable
(xi) SEQUENCE DESCRIPTION: SEQ ID NO:2:

### 4) INFORMATION FOR SEQ ID NO:3:

(i) SEQUENCE CHARACTERISTICS:
   (A) LENGTH: 133 amino acids
   (B) TYPE: amino acid
   (C) STRANDEDNESS: Not Applicable
   (D) TOPOLOGY: linear
(ii) MOLECULE TYPE: peptide
(iii) HYPOTHETICAL: No
(iv) ANTI-SENSE: Not Applicable
(v) FRAGMENT TYPE: Not Applicable
(vi) ORIGINAL SOURCE:
   (A) ORGANISM: Betula verrucosa
(vii) IMMEDIATE SOURCE: Not applicable
(viii) POSITION IN GENOME: Not applicable
(ix) FEATURE:
   (D) OTHER INFORMATION: Amino acid sequence identity with profilin of other organisms is as follows: 30% with human profilin, 28% with calf and mouse, 26% with yeast and 25% with Acanthamoeba
(x) PUBLICATION INFORMATION: Not Applicable
(xi) SEQUENCE DESCRIPTION: SEQ ID NO:3:
   Met Ser Trp Gln Thr Tyr Val Asp Glu His Leu Met Cys Asp Ile Asp
    1               5                   10                  15
   Gly Gln Ala Ser Asn Ser Leu Ala Ser Ala Ile Val Gly His Asp Gly
               20                  25                  30
   Ser Val Trp Ala Gln Ser Ser Ser Phe Pro Gln Phe Lys Pro Gln Glu
           35                  40                  45
   Ile Thr Gly Ile Met Lys Asp Phe Glu Glu Pro Gly His Leu Ala Pro
       50                  55                  60
   Thr Gly Leu His Leu Gly Gly Ile Lys Tyr Met Val Ile Gln Gly Glu
   65                  70                  75                  80
   Ala Gly Ala Val Ile Arg Gly Lys Lys Gly Ser Gly Gly Ile Thr Ile
                   85                  90                  95
   Lys Lys Thr Gly Gln Ala Leu Val Phe Gly Ile Tyr Glu Glu Pro Val
               100                 105                 110
   Thr Pro Gly Gln Cys Asn Met Val Val Glu Arg Leu Gly Asp Tyr Leu
           115                 120                 125
   Ile Asp Gln Gly Leu
       130

### (5) INFORMATION FOR SEQ ID NO:4:

(i) SEQUENCE CHARACTERISTICS:
   (A) LENGTH: 140 amino acids
   (B) TYPE: amino acid
   (D) TOPOLOGY: linear
(ii) MOLECULE TYPE: peptide
(iii) HYPOTHETICAL: No
(iv) ANTI-SENSE: Not applicable
(v) FRAGMENT TYPE: Not Applicable
(vi) ORIGINAL SOURCE:
   (A) ORGANISM: MOUSE (Murine)
(vii) IMMEDIATE SOURCE: Not applicable
(viii) POSITION IN GENOME: Not applicable
(ix) FEATURE:
   (D) OTHER INFORMATION: 28% identical with the P14 allergen of birch (Betula verrucosa)
(x) PUBLICATION INFORMATION: ref. 12
(xi) SEQUENCE DESCRIPTION: SEQ ID NO:4:
   Met Ala Gly Trp Asn Ala Tyr Ile Asp Ser Leu Met Ala Asp Gly Thr
    1               5                  10                  15
   Cys Gln Asp Ala Ala Ile Val Gly Tyr Lys Asp Ser Pro Ser Val Trp
               20                  25                  30
   Ala Ala Val Pro Gly Lys Thr Phe Val Ser Ile Thr Pro Ala Glu Val
           35                  40                  45
   Gly Val Leu Val Gly Lys Asp Arg Ser Ser Phe Phe Val Asn Gly Leu
       50                  55                  60
   Thr Leu Gly Gly Gln Lys Cys Ser Val Ile Arg Asp Ser Leu Leu Gln
   65                  70                  75                  80
   Asp Gly Glu Phe Thr Met Asp Leu Arg Thr Lys Ser Thr Gly Gly Ala
                   85                  90                  95
   Pro Thr Phe Asn Val Thr Val Thr Met Thr Ala Lys Thr Leu Val Leu
               100                 105                 110
   Leu Met Gly Lys Glu Gly Val His Gly Gly Leu Ile Asn Lys Lys Cys
           115                 120                 125
   Tyr Glu Met Ala Ser His Leu Arg Arg Ser Gln Tyr
       130                 135                 140

### (6) INFORMATION FOR SEQ ID NO:5:

(i) SEQUENCE CHARACTERISTICS:
   (A) LENGTH: 139 amino acids
   (B) TYPE: amino acid
   (D) TOPOLOGY: linear
(ii) MOLECULE TYPE: Peptide
(iii) HYPOTHETICAL: No
(iv) ANTI-SENSE: Not applicable
(v) FRAGMENT TYPE: Not Applicable
(vi) ORIGINAL SOURCE:
   (A) ORGANISM: Calf (Bovine)
(vii) IMMEDIATE SOURCE: Not applicable
(viii) POSITION IN GENOME: Not applicable
(ix) FEATURE:
   (D) OTHER INFORMATION: 28% identical with the P14 allergen of birch (Betula verrucosa)
(x) PUBLICATION INFORMATION: ref.14
(xi) SEQUENCE DESCRIPTION: SEQ ID NO:5:
   Ala Gly Trp Asn Ala Tyr Ile Asp Asn Leu Met Ala Asp Gly Thr Cys
    1               5                  10                  15
   Gln Asp Ala Ala Ile Val Gly Tyr Lys Asp Ser Pro Ser Val Trp Ala
               20                  25                  30
   Ala Val Pro Gly Lys Thr Phe Val Asn Ile Thr Pro Ala Glu Val Gly
           35                  40                  45
   Ile Leu Val Gly Lys Asp Arg Ser Ser Phe Phe Val Asn Gly Leu Thr
       50                  55                  60
   Leu Gly Gly Gln Lys Cys Ser Val Ile Arg Asp Ser Leu Leu Gln Asp
    65                  70                  75                  80
   Gly Glu Phe Thr Met Asp Leu Arg Thr Lys Ser Thr Gly Gly Ala Pro
                    85                  90                  95
   Thr Phe Asn Ile Thr Val Thr Met Thr Ala Lys Thr Leu Val Leu Leu
               100                 105                 110
   Met Gly Lys Gln Gly Val His Gly Gly Met Ile Asn Lys Lys Cys Tyr
           115                 120                 125
   Glu Met Ala Ser His Leu Arg Arg Ser Gln Tyr
       130                 135

### (7) INFORMATION FOR SEQ ID NO:6

(i) SEQUENCE CHARACTERISTICS:
   (A) LENGTH: 140 amino acids
   (B) TYPE: amino acid
   (D) TOPOLOGY: linear
(ii) MOLECULE TYPE: Peptide
(iii) HYPOTHETICAL: No
(iv) ANTI-SENSE: Not applicable
(v) FRAGMENT TYPE: Not Applicable
(vi) ORIGINAL SOURCE:
   (A) ORGANISM: Human (Homo sapiens)
(vii) IMMEDIATE SOURCE: Not applicable
(viii) POSITION IN GENOME: Not applicable
(ix) FEATURE:
   (D) OTHER INFORMATION: 30% identical with the P14 allergen of birch (Betula verrucosa)
(x) PUBLICATION INFORMATION: ref. 13
(xi) SEQUENCE DESCRIPTION: SEQ ID NO:6:

### (8) INFORMATION FOR SEQ ID NO:7:

(i) SEQUENCE CHARACTERISTICS:
   (A) LENGTH: 126 amino acids
   (B) TYPE: amino acid
   (D) TOPOLOGY: linear
(ii) MOLECULE TYPE: Peptide
(iii) HYPOTHETICAL: No
(iv) ANTI-SENSE: Not applicable
(v) FRAGMENT TYPE: Not applicable
(vi) ORIGINAL SOURCE:
   (A) ORGANISM: Yeast
(vii) IMMEDIATE SOURCE: Not applicable
(viii) POSITION IN GENOME: Not applicable
(ix) FEATURE:
   (D) OTHER INFORMATION: 26% identical with the P14 allergen of birch (Betula verrucosa)
(x) PUBLICATION INFORMATION: ref. 11
(xi) SEQUENCE DESCRIPTION: SEQ ID NO:7:
   Met Ser Trp Gln Ala Tyr Thr Asp Asn Leu Ile Gly Thr Gly Lys Val
    1              5                  10                  15
   Asp Lys Ala Val Ile Tyr Ser Arg Ala Gly Asp Ala Val Trp Ala Thr
               20                  25                  30
   Ser Gly Gly Leu Ser Leu Gln Pro Asn Glu Ile Gly Glu Ile Val Gln
           35                  40                  45
   Gly Phe Asp Asn Pro Ala Gly Leu Gln Ser Asn Gly Leu His Ile Gln
       50                  55                  60
   Gly Gln Lys Phe Met Leu Leu Arg Ala Asp Asp Arg Ser Ile Tyr Gly
   65                  70                  75                  80
   Arg His Asp Ala Glu Gly Val Val Cys Val Arg Thr Lys Gln Thr Val
                   85                  90                  95
   Ile Ile Ala His Tyr Pro Pro Thr Val Gln Ala Gly Glu Ala Thr Lys
               100                 105                 110
   Ile Val Glu Gln Leu Ala Asp Tyr Leu Ile Gly Val Gln Tyr
           115                 120                 125

### (9) INFORMATION FOR SEQ ID NO:8:

(i) SEQUENCE CHARACTERISTICS:
   (A) LENGTH: 125 amino acids
   (B) TYPE: amino acid
   (D) TOPOLOGY: linear
(ii) MOLECULE TYPE: Peptide
(iii) HYPOTHETICAL: No
(iv) ANTI-SENSE: Not applicable
(v) FRAGMENT TYPE: Not Applicable
(vi) ORIGINAL SOURCE:
   (A) ORGANISM: Acanthamoeba
(vii) IMMEDIATE SOURCE: Not applicable
(viii) POSITION IN GENOME: Not applicable
(ix) FEATURE:
   (D) OTHER INFORMATION: 25% identical with the P14 allergen of birch (Betula verrucosa)
(x) PUBLICATION INFORMATION: ref. 10
(xi) SEQUENCE DESCRIPTION: SEQ ID NO:8:
   Thr Trp Gln Ser Tyr Val Asp Thr Asn Leu Val Gly Thr Gly Ala Val
    1              5                  10                  15
   Thr Gln Ala Ala Ile Leu Gly Leu Asp Gly Asn Thr Trp Ala Ser Phe
               20                  25                  30
   Ala Gly Phe Ala Val Thr Pro Ala Gln Gly Thr Thr Leu Ala Gly Ala
           35                  40                  45
   Phe Asn Asn Ala Asp Ala Ile Arg Ala Gly Gly Phe Asp Leu Ala Gly
        50                  55                  60
   Val His Tyr Val Thr Leu Arg Ala Asp Asp Arg Ser Ile Tyr Gly Lys
    65                  70                  75                  80
   Lys Gly Ala Ser Gly Val Ile Thr Val Lys Thr Ser Lys Ser Ile Leu
                    85                  90                  95
   Val Gly Val Tyr Asn Glu Lys Ile Gln Pro Gly Thr Ala Ala Asn Val
               100                 105                 110
   Val Glu Lys Leu Ala Asp Tyr Leu Ile Gly Gln Gly Phe
           115                 120                 125

### (10) INFORMATION FOR SEQ ID NO: 9:

(i) SEQUENCE CHARACTERISTICS:
   (A) LENGTH: 641 nucleotides
   (B) TYPE: nucleic acid
   (C) STRANDEDNESS: single
   (D) TOPOLOGY: linear
(ii) MOLECULE TYPE: cDNA of mRNA
(iii) HYPOTHETICAL: No
(iv) ANTI-SENSE: No
(v) FRAGMENT TYPE: Not applicable
(vi) ORIGINAL SOURCE:
   (A) ORGANISM: Phleum pratense
(vii) IMMEDIATE SOURCE:
   (A) Pollen from Allergon AB, Engelholm, Sweden
(viii) POSITION IN GENOME: Not applicable
(ix) FEATURE: Not applicable
(x) PUBLICATION INFORMATION: Not applicable
(xi) SEQUENCE DESCRIPTION: SEQ ID NO: 9:
   GAAAGCAAACTTGCAGGACCGAAGATGTCGTGGCAGACGTACGTGGACGAGCACCTGATG  60
   TGCGAGATCGAGGGCCACCACCTCGCCTCGGCGGCCATCCTCGGCCACGACGGCACCGTC 120
   TGGGCCCAGAGCGCCGACTTCCCCCAGTTCAAGCCTGAGGAGATCACCGGCATCATGAAG 180
   GATTTCGACGAGCCGGGGCACCTCGCCCCCACCGGCATGTTCGTCGCAGGTGCCAAGTAC 240
   ATGGTCATCCAGGGTGAACCCGGTCGCGTCATCCGTGGCAAGAAGGGAGCAGGAGGCATC  300
   ACCATAAAGAAGACCGGGCAGGCGCTGGTCGTCGGCATCTATGACGAGCCCATGACCCCT 360
   GGGCAGTGCAACATGGTGGTGGAGAGGCTTGGCGACTACCTCGTTGAACAAGGCATGTAG 420
   ACTGGCTGATCCATGGCTTCCACGTCTCCACGATCGATGATGATCATACAGTTTTTCACG 480
   TTCTTTTAAACATCTATTGGAATATATATGGGGCTTCTCCTCTTTTACCGGCTCTGGTCA 540
   TGGATCACTGATGACCAGTTGCTCTGGAAGTTTCATTTGTAATGCCATCTTGGCTTTCTA 600
   TCTTCTTCAATGTTTTTTTTTTCTTTTCGGTTAAAAAAAAA                    641

### (11) INFORMATION FOR SEQ ID NO: 10:

(i) SEQUENCE CHARACTERISTICS:
   (A) LENGTH: 393 nucleotides
   (B) TYPE: nucleic acid
   (C) STRANDEDNESS: single
   (D) TOPOLOGY: linear
(ii) MOLECULE TYPE: cDNA of mRNA, coding region
(iii) HYPOTHETICAL: No
(iv) ANTI-SENSE: No
(v) FRAGMENT TYPE: Not applicable
(vi) ORIGINAL SOURCE:
   (A) ORGANISM: Phleum pratense
(vii) IMMEDIATE SOURCE:
   (A) Pollen from Allergon AB, Engelholm, Sweden
(viii) POSITION IN GENOME: Not applicable
(ix) FEATURE: Not applicable
(x) PUBLICATION INFORMATION: Not applicable
(xi) SEQUENCE DESCRIPTION: SEQ ID NO: 10:
   ATGTCGTGGCAGACGTACGTGGACGAGCACCTGATGTGCGAGATCGAGGGCCACCACCTC  60
   GCCTCGGCGGCCATCCTCGGCCACGACGGCACCGTCTGGGCCCAGAGCGCCGACTTCCCC 120
   CAGTTCAAGCCTGAGGAGATCACCGGCATCATGAAGGATTTCGACGAGCCGGGGCACCTC 180
   GCCCCCACCGGCATGTTCGTCGCAGGTGCCAAGTACATGGTCATCCAGGGTGAACCCGGT 240
   CGCGTCATCCGTGGCAAGAAGGGAGCAGGAGGCATCACCATAAAGAAGACCGGGCAGGCG 300
   CTGGTCGTCGGCATCTATGACGAGCCCATGACCCCTGGGCAGTGCAACATGGTGGTGGAG 360
   AGGCTTGGCGACTACCTCGTTGAACAAGGCATG                            393

### (12) INFORMATION FOR SEQ ID NO: 11:

(i) SEQUENCE CHARACTERISTICS:
   (A) LENGTH: 131 amino acid residues
   (B) TYPE: amino acid
   (C) STRANDEDNESS: Not applicable
   (D) TOPOLOGY: linear
(ii) MOLECULE TYPE: peptide, P14(T) allergen
(iii) HYPOTHETICAL: No
(iv) ANTI-SENSE: Not applicable
(v) FRAGMENT TYPE: Not applicable
(vi) ORIGINAL SOURCE:
   (A) ORGANISM: Phleum pratense
(vii) IMMEDIATE SOURCE: Not applicable
(viii) POSITION IN GENOME: Not applicable
(ix) FEATURE: Amino acid identity with P14 allergen from Betula verrucosa is 77%
(x) PUBLICATION INFORMATION: Not applicable
(xi) SEQUENCE DESCRIPTION: SEQ ID NO: 11: